# EUROPÄISCHE PATENTSCHRIFT

(11) **EP 0 807 103 B1**
(45) Veröffentlichungstag und Bekanntmachung des Hinweises auf die Patenterteilung: **07.04.1999**
(21) Anmeldenummer: 96902248.2
(22) Anmeldetag: 23.01.1996
(51) Int. Cl.: C07C 305/06, C11D 1/16

(54) **DIMERALKOHOL-BIS UND TRIMERALKOHOL-TRIS-SULFATE UND -ETHERSULFATE**
DIMERIC ALCOHOL-BIS AND TRIMERIC ALCOHOL-TRIS-SULPHATES AND ETHER SULPHATES
SULFATES ET ETHERS-SULFATES D'ALCOOLS DIMERES BIS ET D'ALCOOLS TRIMERES TRIS

(30) Priorität: 01.02.1995 DE 19503061
(43) Veröffentlichungstag der Anmeldung: 19.11.1997
(73) Patentinhaber: Henkel Kommanditgesellschaft auf Aktien, 40191 Düsseldorf (DE)
(72) Erfinder: KNÜBEL, Georg, D-40229 Düsseldorf (DE); WESTFECHTEL, Alfred, D-40723 Hilden (DE); HERMANNS, Gerhard, D-40789 Monheim (DE); HOFMANN, Rainer, D-40597 Düsseldorf (DE); RATHS, Hans-Christian, D-40789 Monheim (DE)
(86) Internationale Anmeldenummer: EP9600259
(87) Internationale Veröffentlichungsnummer: WO9623768

(56) Entgegenhaltungen:
- EP-A- 0 247 467
- EP-A- 0 401 642
- US-A- 1 968 797

## Beschreibung

Die Erfindung betrifft Dimeralkohol-bis und Trimeralkohol-tris-sulfate und -ethersulfate, ein Verfahren zu ihrer Herstellung durch Umsetzung von Dimer- und Trimeralkoholen mit Sulfiermitteln und anschließender Neutralisation sowie ihre Verwendung als oberflächenaktive Substanzen und als Pastenverflüssiger.

### Stand der Technik

Unter Gemini-Tensiden sind solche Verbindungen zu verstehen, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, daß die hydrophilen Gruppen einen ausreichenden Abstand haben, damit sie unabhängig voneinander agieren können. (vgl. **M. Rosen, Chemtech, March, (1993), S.30 ff; F. Menger, C.A.Littau, J. Am. Chem. Soc. 115 (1993), S. 10083 ff.).**

Die in den genannten Literaturstellen beschriebenen Tenside zeichnen sich durch eine ungewöhnlich geringe kritische Micellkonzentration aus und die Fähigkeit, die Oberflächenspannung des Wassers stark zu reduzieren. Diese Tenside sind jedoch synthetisch nur schwer, teilweise durch mehrstufige Synthesen, zugänglich. Die aufwendigen Synthesewege führen zwangsläufig zu höheren Kosten, was für eine technische Anwendung und Nutzung in industriellem Maßstab, nachteilig ist.

Der vorliegenden Erfindung lag die Aufgabe zugrunde, ein Verfahren zur Herstellung von neuen Gemini-Tensiden zur Verfügung zu stellen, wodurch die Tenside in hohen Ausbeuten und auf eine einfache und kostengünstige Weise erhalten werden können.

### Gegenstand der Erfindung

Gegenstand der vorliegenden Erfindung sind Dimeralkohol-bis-sulfate und Trimeralkohol-tris-sulfate und -ethersulfate, die dadurch erhältlich sind, daß man Dimeralkohole und/oder Trimeralkohole bzw. deren Anlagerungsprodukte mit Alkylenoxiden mit einem Sulfiermittel umsetzt und anschließend mit einer wäßrigen Base neutralisiert.

Überraschenderweise wurde festgestellt, daß bei der Sulfatierung von Dimer- und Trimeralkoholen bzw. deren Alkoxylaten die entsprechenden Bis-sulfate und Tris-sulfate in guter Ausbeute und hohen Sulfiergraden erhalten werden. Die erhaltenen Verbindungen sind sogenannte Gemini-Tenside, wobei die beiden anionischen Sulfatgruppen in der Regel in einem Abstand von etwa 18 Kohlenstoffatomen angeordnet sind, was gewährleistet, daß diese beiden hydrophilen Gruppen unabhängig voneinander agieren können.

Ein weiterer Gegenstand der vorliegenden Erfindung ist ein Verfahren zur Herstellung von Dimeralkohol-bis-sulfaten und Trimeralkohol-tris-sulfaten und -ethersulfaten, das sich dadurch auszeichnet, daß man Dimeralkohole und/oder Trimeralkohole bzw. deren Anlagerungsprodukte mit Alkylenoxiden mit einem Sulfiermittel umsetzt und anschließend mit einer wäßrigen Base neutralisiert.

### Dimer- und Trimeralkohole

Die erfindungsgemäß eingesetzten Dimer- und Trimeralkohole sind im Handel erhältliche Verbindungen und können beispielsweise durch Reduktion von Dimer- und Trimerfettsäureestern gewonnen werden. Die Dimer- und Trimerfettsäuren selbst können z.B. durch Oligomerisierung von ungesättigten Fettsäuren erhalten werden. Die Dimer- und Trimerfettsäuren sind in der Regel Gemische aus acyclischen und cyclischen Dicarbonsäuren mit durchschnittlich 36 bis 44 Kohlenstoffatomen [vgl. **A. Hinze in Fette & Öle,26 (1994)].**

### Dimer- und Trimeralkaholalkoxylate

Die als Ausgangsverbindungen eingesetzten Dimer- und Trimeralkoholalkoxylate können in an sich bekannter Weise durch Alkoxylierung der Dimer- und Trimeralkohole erhalten werden. Zu den bevorzugt eingesetzten Alkoxylaten gehören beispielsweise die Ethoxylate und die Propoxylate oder Addukte die sowohl Ethoxyals auch Propoxygruppen im Molekül enthalten. Besonders bevorzugt werden Addukte mit durchschnittlich 1 bis 20 Mol Ethylenoxid pro OH-Gruppe eingesetzt, die gegebenenfalls auch durchschnittlich 1 bis 5 Mol Propylenoxid pro Mol Alkohol enthalten können. Die angegebenen Alkoxylierungsgrade stellen statistische Mittelwerte dar, die für ein spezielles Produkt eine ganze oder eine gebrochene Zahl sein können. Bevorzugte Dimer- und Trimeralkoholalkoxylate weisen eine eingeengte Homologenverteilung auf (narrow range ethoxylates, NRE).

Zur Durchführung des erfindungsgemäßen Verfahrens werden die Dimer- und Trimeralkohole bzw. deren Alkoxylate üblicherweise in einem Reaktionsreaktor vorgelegt. Die Umsetzung zum Dimer- oder Trimeralkohol-bis-sulfat kann dann durch kontinuierliches Einbringen des Sulfiermittels erfolgen.

Die Sulfatierung der Dimer- und Trimeralkohole bzw. deren Alkoxylate kann man mit üblichen Sulfiermitteln, wie z.B. Schwefelsäure, Oleum, Chlorsulfonsäure, Aminosulfonsäure und gasförmigem Schwefeltrioxid im Gemisch mit einem Inertgas erfolgen, wobei Chlorsulfonsäure und gasförmiges Schwefeltrioxid bevorzugt sind. Beim Einsatz von Schwefeltrioxid wird dieses mit Luft oder Stickstoff verdünnt und vorzugsweise wird ein Gasgemisch mit ca. 1 bis 8, insbesondere 3 bis 5 Vol.-% Schwefeltrioxid eingesetzt.

Das molare Einsatzverhältnis von OH-Gruppen der Ausgangsverbindungen zu Sulfiermittel kann 1 : 0,95 bis 1 : 1,8 und vorzugsweise 1 : 1,0 bis 1 : 1,3 betragen. Üblicherweise wird die Sulfatierung bei 20 bis 98°C durchgeführt. Im Hinblick auf die Viskosität der Ausgangsverbindungen einerseits und die Farbqualität der resultierenden Sulfatierungsprodukte andererseits, hat sich ein Temperaturbereich von 25 bis 70°C als optimal erwiesen.

Das bei der Sulfatierung anfallende saure rohe sulfatierte Produkt wird anschließend mit einer Base neutralisiert und bevorzugt auf einen pH-Wert unter 9, besonders bevorzugt von 6,5 bis 8,5 eingestellt. Ein pH-Wert über 9,5 sollte vermieden werden. Es wurde festgestellt, daß bei sehr hohen pH-Werten die Ester bindung instabil ist und bereits bei einer kurzfristigen Überschreitung eine starke Verseifung zu beobachten ist. Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen werden bevorzugt in Form 5 bis 55 gew.-%iger wäßriger Lösungen eingesetzt, wobei 5 bis 25 gew.-%ige wäßrige Natriumhydroxidlösungen bevorzugt sind.

Die Sulfatierungsprodukte können nach der Neutralisation in an sich bekannter Weise durch Zusatz von Wasserstoffperoxid- oder Natriumhypochloridlösung gebleicht werden. Dabei werden, bezogen auf den Feststoffgehalt der Sulfatierungsprodukte in der Lösung, 0,2 bis 2 Gew.-% Wasserstoffperoxid, berechnet als 100 Gew.-%ige Substanz oder entsprechende Mengen Natriumhypochlorid eingesetzt. Der pH-Wert der Lösungen kann unter Verwendung geeigneter Puffermittel, z. B. mit Natriumphosphat oder Zitronensäure konstant gehalten werden. Zur Stabilisierung gegen Bakterienbefall empfiehlt sich ferner eine Konservierung, z. B mit Formaldehydlösung, p-Hydroxybenzoat, Sorbinsäure oder anderen bekannten Konservierungsstoffen.

### Gewerbliche Anwendbarkeit

Die erfindungsgemäßen Dimer-bis-sulfate und Trimeralkohol-tris-sulfate bzw. -ethersulfate weisen grenzflächenaktive Eigenschaften auf. Sie fördern beispielsweise die Benetzung fester Oberflächen und die Emulgierung von ansonsten nicht miteinander mischbaren Phasen.

Ein weiterer Gegenstand der Erfindung betrifft daher die Verwendung der erfindungsgemäßen Verbindungen zur Herstellung von Wasch-, Spül-, Reinigungs- und Avivagemitteln sowie Mitteln zur Haar- und Körperpflege, in denen sie in Mengen von 1 bis 50, vorzugsweise 5 bis 30 Gew.-%, bezogen auf die Mittel, die enthalten sein können.

Außerdem wurde festgestellt, daß sie gut als Pastenverflüssiger für hochviskose Tensidsysteme geeignet sind. Hochviskose Tensidsysteme, z. B. flüssige Tensidpasten mit einem Tensidgehalt von über 40 Gew.-% sind üblicherweise dickflüssige Pasten. Durch Zusatz der erfindungsgemäßen Verbindungen zu derartigen Tensidpasten konnte eine deutliche Verbesserung des Fließverhaltens beobachtet werden.

Demgemäß betrifft ein weiterer Gegenstand die Verwendung der erfindungsgemäßen Verbindungen als Pastenverflüssiger, insbesondere für hochviskose Tensidsysteme. Bevorzugt werden die erfindungsgemäßen Verbindungen den Tensidpasten in einer Menge von 1 Gew.-% bis 10 Gew.-%, bezogen auf das Gewicht der Tensidpaste, zugesetzt. Werden die erfindungsgemäßen Verbindungen in einer Menge unter 0,5 Gew. % eingesetzt so wird in der Regel keine ausreichende Verbesserung des Fließverhaltens erreicht.

Die folgenden Beispiele sollen den Gegenstand der Erfindung näher erläutern.

### Beispiele

### Herstellung von C36-Dimeralkohol-Bissulfat (Di-Natriumsalz)

### Allgemeine Arbeitsvorschrift:

In einem Labor-Conti-Fallfilmreaktor (Reaktorabmessungen: 110 cm Länge, Innendurchmesser: 0,6 cm) mit vorgeschaltetem Oleumverdampfer wurde unter Verwendung eines Stickstoffstromes von ca. 750 l/h ein Produktstrom von 10 g/min eingeführt (Das virtuelle Molekulargewicht wurde dabei aus der OH-Zahl des Ausgangsproduktes bestimmt). Gleichzeitig wurde 65 Gew.-%iges Oleum mit einer Rate von 1,05 Moläquivalenten pro OH-Gruppe in den Verdampfer gepumpt. Das dort entstehende Schwefeltrioxid wurde mit einem Stickstoffstrom von 250 l/h aus dem Verdampfer ausgetrieben und ebenfalls in den Reaktor überführt. Der Reaktor wurde dabei mit Wasser (30 °C) temperiert. Der entstehende Schwefelsäurehalbester wurde am Reaktorausgang entgast und in einem Becherglas unter kontinuierlicher Zugabe und unter Eiskühlung und kräftigem Rühren mit 25 Gew.-%iger Natronlauge neutralisiert.

Die Kenndaten der so erhaltenen Tensidpasten wurden nach den Einheitsmethoden der DGF bestimmt und sind bei den einzelnen Verbindungen aufgeführt. Zwecks weiterer analytischer Untersuchungen wurden die Tensidpasten im Vakuum gefriergetrocknet.

### Sulfatierung von Dimeralkohol

Als Ausgangsverbindung wurde Sovermol RPOL 900 (ein C-36-Dimeralkohol; Handelsprodukt der Fa. Henkel, Düsseldorf) auf eine Temperatur von 80 °C vorerwärmt, um das Ausgangsprodukt aufzuschmelzen. Nach beendeter Reaktion wurde das Reaktionsprodukt noch zwei Stunden im Dampfbad nachhydrolisiert, der pH-Wert wurde dabei mit einigen Tropfen NaOH auf 7 bis 9 gehalten.

### Kenndaten des C36-Dimeralkohol-Bissulfates (Di-Natriumsalz):

| | |
|---|---|
| Trockenrückstand (TR) | 31 Gew.-% |
| Unsulfierte Anteile (US) | 1,2 Gew.-% |
| Sulfiergrad (S °) | 96 % |
| Natriumsulfat | 1,2 Gew.-% |
| Organisch gebundener Schwefel | 2,2 Gew.-% (entspricht 2,1 Mol S pro Mol Disulfat) |

### Verwendung des C36-Dimeralkohol-Bissulfates (Di-Natriumsalz) als Pastenverflüssiger:

Die Wirkung der Fließverbesserung wurde durch Zusatz der erhaltenen Verbindung zu einer Tensidpasten, die 65 Gew.-% C₁₂-C₁₄-Fettalkohol x 2 EO-sulfat-Na-Salz enthielt, bei 25°C untersucht.

Die in der nachfolgenden Tabelle dargestellten Ergebnisse zeigen, daß schon bei einem geringen Zusatz von 1 Gew.-% der erfindungsgemäßen Verbindung eine deutliche Verbesserung der Fließeigenschaften der Tensidpaste erhalten wird.

## Patentansprüche

1. Dimeralkohol-bis-sulfate und Trimeralkohol-tris-sulfate und -ethersulfate, die dadurch erhältlich sind, daß man Dimeralkohole und/oder Trimeralkohole bzw. deren Alnlagerungsprodukte mit Alkylenoxiden mit einem Sulfiermittel umsetzt und anschließend mit einer wäßrigen Base neutralisiert.

2. Verfahren zur Herstellung von Dimeralkohol-bis-sulfaten und Trimeralkohol-tris-sulfaten und -ethersulfaten, **dadurch gekennzeichnet, daß** man Dimeralkohole und/oder Trimeralkohole bzw. deren Alnlagerungsprodukte mit Alkylenoxiden mit einem Sulfiermittel umsetzt und anschließend mit einer wäßrigen Base neutralisiert.

3. Verfahren nach Anspruch 2, **dadurch gekennzeichnet, daß** man als Alkohle Dimeralkohole mit durchschnittlich 36 bis 44 Kohlenstoffatomen einsetzt.

4. Verfahren nach Anspruch 2 oder 3, **dadurch gekennzeichnet, daß** man als Sulfiermittel Chlorsulfonsäure oder gasförmiges Schwefeltrioxid einsetzt.

5. Verfahren nach einem der Ansprüche 1 bis 4, **dadurch gekennzeichnet, daß** man die Sulfatierung mit einem molaren Verhältnis pro OH-Gruppe von Dimer- bzw. Trimeralkohol bzw. des -alkoxylats zu Sulfiermittel von 1 : 0,95 bis 1 : 1,8 durchführt.

6. Verfahren nach einem der Ansprüche 1 bis 5, **dadurch gekennzeichnet, daß** man die Sulfierung bei Temperaturen von 50 bis 98°C durchführt.

7. Verfahren nach einem der Ansprüche 1 bis 6, **dadurch gekennzeichnet, daß** man die Neutralisation mit 5 bis 55 gew.-%igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen gebildeten Gruppe durchführt.

8. Verwendung der Dimer-bis-sulfaten und Trimeralkohol-tris-sulfate und -ethersulfate nach Anspruch 1 zur Herstellung von Wasch-, Spül-, Reinigungs- und Avivagemitteln sowie Mitteln zur Haar- und Körperpflege.

9. Verwendung der Dimer-bis-sulfaten und Trimeralkohol-tris-sulfate und -ethersulfate nach Anspruch 1 als Pastenverflüssiger.

## Claims

1. Dimer alcohol bis-sulfates and trimer alcohol tris-sulfates and ether sulfates which are obtainable by reaction of dimer alcohols and/or trimer alcohols or addition products thereof with alkylene oxides with a sulfating agent and subsequent neutralization with an aqueous base.

2. A process for the production of dimer alcohol bis-sulfates and trimer alcohol tris-sulfates and ether sulfates, characterized in that dimer alcohols and/or trimer alcohols or addition products thereof with alkylene oxides are reacted with a sulfating agent and the product of this reaction is neutralized with an aqueous base.

3. A process as claimed in claim 2, characterized in that dimer alcohols containing an average of 36 to 44 carbon atoms are used as the alcohols.

4. A process as claimed in claim 2 or 3, characterized in that chlorosulfonic acid or gaseous sulfur trioxide is used as the sulfating agent.

5. A process as claimed in any of claims 1 to 4, characterized in that the sulfation step is carried out with a molar ratio per OH group of dimer or trimer alcohol or alkoxylate to sulfating agent of 1:0.95 to 1:1.8.

6. A process as claimed in any of claims 1 to 5, characterized in that the sulfation is carried out at temperatures of 50 to 98°C.

7. A process as claimed in any of claims 1 to 6, characterized in that the neutralization step is carried out with 5 to 55% by weight aqueous bases from the group consisting of alkali metal hydroxides, alkaline earth metal oxides and hydroxides, ammonia, mono-, di- and tri-C₂₋₄-alkanolamines and primary, secondary and tertiary C₁₋₄ alkyl amines.

8. The use of the dimer alcohol bis-sulfates and trimer alcohol tris-sulfates and ether sulfates claimed in claim 1 for the production of laundry detergents, dishwashing detergents, cleaners and fabric softeners and also hair-care and body-care formulations.

9. The use of the dimer alcohol bis-sulfates and trimer alcohol tris-sulfates and ether sulfates claimed in claim 1 as liquefiers for pastes.

## Revendications

1. Bis sulfates d'alcool dimère et trisulfates d'alcool trimère et éther-sulfates de ceux-ci, qui sont accessibles en ce qu'
on fait réagir des alcools dimères et/ou des alcools trimères ou leurs produits d'addition avec des oxydes d'alkylène, avec un agent de sulfatation et qu'ensuite on neutralise avec une base aqueuse.

2. Procédé de préparation de bis sulfates d'alcool dimère et de trisulfates d'alcool trimère et des éther-sulfates de ceux-ci,
caractérisé en ce qu'
on fait réagir des alcools dimères et/ou des alcools trimères ou leurs produits d'addition avec des oxydes d'alkylène avec un agent de sulfatation et qu'ensuite on neutralise avec une base aqueuse.

3. Procédé selon la revendication 2,
caractérisé en ce qu'
on met en oeuvre comme alcools des alcools dimères ayant en moyenne 36 à 44 atomes de carbone

4. Procédé selon la revendication 2 ou la revendication 3,
caractérisé en ce qu'
on met en oeuvre comme agent de sulfatation de l'acide chlorosulfonique ou de l'anhydride sulfurique gazeux.

5. Procédé selon l'une des revendications 1 à 4,
caractérisé en ce qu'
on effectue la sulfatation avec un rapport molaire par groupe OH de l'alcool dimère ou trimère ou de l'alcoxylate d'alcool dimère ou trimère à l'agent de sulfatation, allant de 1:0,95 à 1:1,8.

6. Procédé selon l'une des revendications 1 à 5,
caractérisé en ce qu'
on effectue la sulfatation à des températures allant de 50 à 98°C.

7. Procédé selon l'une des revendications 1 à 6,
caractérisé en ce qu'
on effectue la neutralisation avec des bases aqueuses de 5 à 55 % en poids, choisies dans le groupe formé par les hydroxydes de métal alcalin, les oxydes et les hydroxydes de métal alcaline-terreux, l'ammoniac, les mono, di et tri(alcanol en C₂-C₄) amines ainsi que les (alkyl en C₁-C₄) amines primaires, secondaires et tertiaires.

8. Utilisation des bis sulfates d'alcool dimère, des trisulfates d'alcool trimère et des sulfates d'éther de ceux-ci selon la revendication 1, pour la production de produits de lavage, de rinçage, de nettoyage et d'avivage ainsi que de produits pour le soin des cheveux et du corps.

9. Utilisation des bis sulfates de dimère, et de trisulfates de trimères et de sulfates d'éther selon la revendication 1 en tant que produit de liquéfaction de pâtes.
